# EUROPEAN PATENT APPLICATION

(11) **EP 1 369 482 A1**
(43) Date of publication of application: **10.12.2003**
(21) Application number: 02012705.6
(22) Date of filing: 07.06.2002
(51) Int. Cl.: C12N 15/12, C07K 14/47, C07K 16/18, C12N 5/10, A61K 31/00, A61K 38/00, A61K 48/00, A61P 35/00, G01N 33/53

(54) **Target genes for the diagnosis and treatment of cancer**

(71) Applicant: Deutsches Krebsforschungszentrum Stiftung des öffentlichen Rechts, 69120 Heidelberg (DE)
(72) Inventor: Wittig, Rainer, 69121 Heidelberg (DE); Poustka, Annemarie, 69120 Heidelberg (DE); Mollenhauer, Jan, 69118 Heidelberg (DE); Schadendorf, Dirk, 68165 Mannheim (DE)
(74) Representative: Isenbruck, Günter, Dr.

(57) **Abstract**

The present invention relates to the identification and use of target genes for the detection and treatment of drug-resistant tumor cells. The nucleic acids of the present invention exhibit a deregulated phenotype when the tumor cells are subjected to cytostatic drugs, i.e. they are expressed in a higher or lower amount as compared to parental drug-sensitive cancer cells. Thus, they can be used as a diagnostic and pharmaceutical tool to render drug-resistant cells drug-sensitive. In addition, the present invention includes the polypeptides encoded by the respective nucleic acids, expression vectors harboring the nucleic acids, host cells for expression and methods for the diagnosis and treatment of drug-resistant tumor cells.

## Description

The present invention relates to the identification and use of target genes for the detection and treatment of drug-resistant tumor cells.

Cancer is the second major cause of death in Europe and Northern America. In view of the increasing number in cancer cases, any approach to improve current treatment methods is of important ethic, social and economic value. The effective cure of patients, though, is often difficult since many tumor cells develop a resistance to anticancer drugs used for chemotherapy. The described phenotype, also termed drug resistance (DR), involves a variety of strategies that tumor cells use to evade the cytostatic effects of anticancer drugs. DR is characterized by a decreased sensitivity of tumor cells to the drug employed for chemotherapy and, often, also to a broad spectrum of further drugs with neither obvious structural homology nor common targets. Mechanisms for DR include modifications in detoxification and DNA repair pathways, changes in cellular sites of drug sequestration, decreases in drug-target affinity, synthesis of specific drug inhibitors within cells, and accelerated removal or secretion of drugs. More recently, cellular drug resistance has been associated with alterations in cell death pathways, a process known as apoptosis, but to date the molecular basis and exact mechanisms remain poorly understood.

Acute or chronic exposure to cancer drugs produces an adaptive response in tumor cells, mainly caused by an altered gene expression as compared to drug-sensitive cells. The altered gene expression found in various human tumors is often caused by the amplification, deletion, recombination, or other mutations of certain genes. Increased or decreased expression of those target genes usually provide a selective advantage for the tumor cells and play therefore an important role for the pathogenesis and prognosis of DR in human cancer.

Changes in gene expression in target cells can lead to an acquired resistant phenotype and can also provide clues about intracellular targets essential for overcoming DR. From the foregoing, it becomes evident that tumor cells which are resistant to anticancer drugs due to the overexpression of particular genes may be rendered drug-sensitive and, thus, treatable if the respective genes, RNAs, or proteins are specifically knocked out, inhibited, or down-regulated. Similarly, drug resistance as a result of little or no expression of particular genes may be overcome by supplementing the function of the underrepresented or missing gene.

The object of the present invention is to provide target genes that are useful for the effective treatment of tumor cells using anticancer drugs.

The present invention is directed to genes, which are differentially expressed in multiple drug-resistant cells relative to drug-sensitive cells. Thus, the object is attained by nucleic acids encoding human polypeptides or immunogenic fragments thereof, which are deregulated in drug-resistant tumor cells in response to drug exposure as compared to the drug-sensitive parental cells, wherein said nucleic acids comprise those genes which are disclosed by the following complete and/or partial sequence identification numbers: U63131, L11667, L48513, Z30093, X13293, L26081, M14200, X91171, D23662, L28824, L12401, D10522, M94345, AF063228, M19267, AF009615, U92436, U59831, U19251, Z50781, X79550, AF035812, X63468, M22919, L19605, X06820, M33336, S74017, L11066, U59877, M19645, X92669, D16581, M81601, V00530, D29013, M29874, L13740, M60974, X79683, U84388, M95809, D49547, L06623, D90209, J05428, K02770, X15880, X76771, X53280, M22760, U04520, L20298, Y14690, U13991, X06994, M22299, U44754, X58531, U84573, U11690, H45315, M11717, X85106, D50310, W68277, R09223, U36798, J04164, R76702, AB000584, R97220, M22976, AB023155, U79303, X67325, AB005047, AA410441, U46499, AB021288, U31345, M54968, M65212, J02611, X90858, U18728, M13755, H00952, H11608, X15729, H57310, M64925, L18967, AK000917, NM_018330, U90878, X02492, U50648, U09510, X01060, X57352, S45630, J04129, S40706, AF126743, NM_015894, NM_013451, AI624266, AL117608, NM_020182, NM_021216, AC007228, pos.66685-66811 (**see Table 1**).

The present invention further includes derivatives and fragments of any of said nucleic acids, provided that their capacity to overcome the said drug resistance remains unaffected. The term derivative or fragment of the disclosed sequences refers to DNA sequences in which one or more nucleotides of the coding sequence can be substituted by one or more nucleotides different from the original one(s), or DNA sequences the nucleotide sequence of which is either extended, shortened, or both, on either the 5'-, or the 3'- or both ends, or in the center portion, provided that the function of the nucleic acids and their encoded proteins remains unaffected.

Furthermore, in the context of the present invention the term derivative or fragment refers to DNA sequences that are complementary to the so-called antisense strand of the disclosed nucleic acids. Hereinafter, they will be further referred to as antisense oligonucleotides.

The present differentially expressed nucleic acids/genes can be identified by methods well known to the person skilled in the art, including comparative PCR, semi-quantitative RT-PCR, immunoscreening, Southern Blotting, Northern blotting, subtractive hybridization, representational difference analysis (RDA), serial analysis of gene expression (SAGE) and mRNA differential display (DD) methods performing a genome-wide scan for differentially expressed genes using cDNA microarray for hybridization studies. The latter represents the preferred method of the present invention.

As used herein, "deregulated" means that the nucleic acids of the present invention are expressed either higher or lower in cells that were exposed to drugs with respect to cells that were not exposed to drugs. In a preferred embodiment of the present invention the term is defined in that the single array spots representing differentially expressed genes display expression ratios of at least 2.5 and 1:2.5 when using the UniGene Array, and expression ratios of at least 1.5 and 1:1.5 when using the drug-resistance subarray (see Materials and Methods).

As used hereinafter, the term "drug", which causes a deregulation of said nucleic acids molecules refers to so-called cytostatic drugs comprising (i) antimetabolites, such as cytarabine, fludarabine, 5-fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, fotemustine, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphteria toxin; (vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-01 (7-hydroxystaurosporine); (x) miscellaneous investigational agents such as thioplatin, PS-341, phenylbutyrate, ET-18-OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; (xi) hormones such as glucocorticoids or fenretinide; (xii) hormone antagonists, such as tamoxifen, finasteride or LHRH antagonists.

In a preferred embodiment of the present invention, the cytostatic drug causing deregulation is selected from the group consisting of cisplatin, etoposide and fotemustine.

The differential, deregulated expression can be achieved by either a loss or a gain of a certain chromosomal region or by other causes leaving the respective chromosomal region unaffected. The determination of potential chromosome changes is done, e.g., by comparative genomic hybridization (CGH), a technique which is known to the person skilled in the art.

Furthermore, the present invention includes the polypeptides, immunogenic fragments and derivatives thereof, encoded by the above mentioned nucleic acids of the present invention, derivatives and fragments thereof, which are deregulated in drug-resistant tumor cells in response to drug exposure as compared to the drug-sensitive parental cells, wherein said polypeptides comprise those which are disclosed by the following sequence identification numbers: AAB04798, AAA35731, AAC41995, CAA82909, CAA31655, AAA65938, AAA52171, CAA62596, BAA04889, AAA36526, NP_005563, BAA01392, AAA59570, AAC33443, AAA36771, AAC51766, AAC51182, AAC50660, AAC52045, CAA90644, CAA56093, AAB88513, CAA45068, AAA59893, AAA19734, CAA29968, AAB50921, AAB32188, NP_004125, AAB02832, AAA52614, CAA63356, BAA04013, AAA61138, CAA23789, BAA06099, AAA52144, AAA36763, AAA35863, CAA56130, AAC50952, AAA58399, BAA08495, AAA52342, BAA14234, AAA36793, AAA36106, CAA33889, CAA54166, CAA37375, AAA99220, AAF66217, AAA02868, CAA75002, AAA62230, CAA30052, AAB02844, AAC50358, CAA41418, AAB58363, AAA57004, NP_003061, AAA52697, CAA59427, BAA08849, NP_002520, NP_000921, AAB18673, AAA35494, NP061885, BAA19151, AAA52133, BAA76782, AAB50227, CAA47739, BAA25922, NP_064696, BAA35182, AAB60371, AAB41942, AAA68927, AAB59517, CAA62369, AAA85268, AAA36038, AAF19251, NP_005505, CAA33751, NP_003059, AAA60059, AAA20870, BAA91423, NP_060800, AAC05580, CAA26322, AAC50768, AAA86443, CAA25527, CAA40626, AAB23453, AAA60147, AAB22646, AAD38506, NP_056978, NP_038479, NP_000912, CAB56012, NP_064567, NP_067039, AAD23608.

In the context of the present invention, the term derivative or fragment of said polypeptides refers to peptides in which one or more aminoacids of the sequences, as disclosed in the above mentioned GenBank numbers, can be substituted by one or more aminoacids different from the original one(s), or peptides the aminoacid sequence of which is either extended, shortened, or both, on either the aminoterminal, or the carboxyterminal or both ends or in the center region with respect to the original proteins, provided that the function of the proteins remains unaffected.

The polypeptides, immunogenic fragments and derivatives thereof, encoded by the above mentioned nucleic acids of the present invention, derivatives and fragments thereof, can be identified by methods well known to the person skilled in the art, including immunoscreening, Western blotting using antibodies directed against the encoded polypeptides, mass spectroscopy to identify differentially expressed polypeptides encoded by the nucleic acids genes, 2D protein gel electrophoresis, protein and antibody arrays to identify differentially expressed polypeptides encoded by the nucleic acids/genes.

The nucleic acids of the present invention and their encoded polypeptides can be used as pharmaceuticals to cure tumor cells that have acquired drug-resistance due to an altered expression pattern of the respective genes as compared to drug-sensitive tumor cells.

If the tumor to be treated is drug-resistant because certain nucleic acids of the present invention are weakly or not expressed as compared to drug-sensitive tumor cells, the tumor can be rendered drug-sensitive, and thus curable, by administering the respective nucleic acids and/or their encoded polypeptides, fragments or derivatives thereof, to supplement for the missing gene activity and lacking protein.

In a further embodiment of the present invention the tumor can be rendered drug-sensitive by administration of so-called "activators" capable of increasing the expression of the weakly or not expressed nucleic acid.

As referred herein, the term "activator" refers to polypeptides, proteins, small molecules, lipids, nucleic acids and derivatives or fragments thereof, that execute their function by either directly binding to the respective nucleic acids, fragments or derivatives thereof, and / or their encoded proteins, fragments or derivatives thereof, or that execute their function indirectly, e.g. by binding to other molecules that increase the expression of the respective nucleic acids. The activator can e.g. act on the level of transcription, translation and all other co- and post-transcriptional and -translational processes as e.g. splicing, translocation, folding, chemical modification, degradation.

Otherwise, if the tumor to be treated is drug-resistant because certain nucleic acids of the present invention are overexpressed as compared to drug-sensitive tumor cells, the tumor can be rendered drug-sensitive, and thus curable, for example by administering antisense oligonucleotides complementary to the respective nucleic acids, fragments or derivatives thereof, to down-regulate gene activity. This method is well known to the person skilled in the art (Vassar R. et al., Science 1999, 286:735-740; Yan R. et al., Nature 1999, 402:533-537).

In a further embodiment of the present invention the tumor can be rendered drug-sensitive by administration of so-called "inhibitors" capable of decreasing the expression of the overexpressed nucleic acid.

As referred herein, the term "inhibitor" refers to polypeptides, proteins, small molecules, lipids, nucleic acids and derivatives or fragments thereof, that execute their function by either directly binding to the respective nucleic acids, fragments or derivatives thereof, and / or their encoded proteins, fragments or derivatives thereof, or that execute their function indirectly, e.g. by binding to other molecules that decrease the expression of the respective nucleic acids. The inhibitor can e.g. act on the level of transcription, translation and all other co- and post-transcriptional and -translational processes as e.g. splicing, translocation, folding, chemical modification, degradation. Inhibitors known to the person skilled in the art include of RNAi molecules, morpholinos, peptide haptamers, antibodies, or small organic or inorganic molecules and compounds.

The nucleic acids as disclosed in the present invention, derivatives and fragments thereof, and the corresponding polypeptides, derivatives and fragments thereof, as well as the described activators and inhibitors, can be used as a pharmaceutical preferably in combination with at least one active compound. This is a further embodiment of the present invention. The term "active compound" refers to a compound other than the disclosed nucleic acids or polypeptides, fragments or derivatives thereof, which is able to induce cell death or which inhibits cell proliferation.

Active compounds which are able to induce cell death are known to the person skilled in the art. In the context of the present invention, said active compounds can be considered to be equivalent to the cytostatic drugs causing deregulation of the nucleic acids of the present invention as specified above.

A further object of the present invention are pharmaceutical preparations which comprise an effective dose of at least one of the disclosed nucleic acids and/or one of the disclosed polypeptides, fragment or derivative thereof, and /or at least one activator or inhibitor, and/or at least one active compound and a pharmaceutically acceptable carrier, i.e. one or more pharmaceutically acceptable carrier substances and/or additives.

The dosage of the nucleic acid, the polypeptide, the activator or the inhibitor in combination with one or more active compounds to be administered, depends on the individual case and is, as is customary, to be adapted to the individual circumstances to achieve an optimum effect.

The nucleic acids, their encoded polypeptides, the activators and the inhibitors according to the present invention, respectively the medicaments containing the latter, can be used for the treatment of all cancer types which are resistant to anticancer/cytostatic drugs due to the deregulation of certain nucleic acids that are included in the present invention. Examples of such cancer types comprise neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, prostate carcinoma and plasmocytoma.

In a preferred embodiment of the invention, the cancer to be analyzed, diagnosed and treated is melanoma.

According to the present invention, the disclosed nucleic acids, fragments or derivatives thereof and/or the disclosed polypeptides, fragments or derivatives thereof can be used as a diagnostic tool to detect aberrant expression in any of said tumor cells suspected of being drug-resistant or in advance to determine the most efficient initial treatment. Moreover, the present genomic nucleic acids can be used to detect expression of homologous mRNAs and to isolate homologous cDNAs.

Nucleic acids homologous to the nucleic acids included in the present invention can be detected by any procedure known to a person skilled in the art. For example, hybridization techniques are frequently used for detecting nucleic acids and the present invention contemplates all available hybridization techniques, including Southern, Northern and in situ hybridization techniques. In particular, Northern hybridization is a preferred method of the present invention to detect homologous nucleic acids.

Hybridization techniques are usually performed by proving a sample of tissue or cells, contacting the sample with a labeled probe, that binds to said nucleic acid molecule, and determining the presence or amount of the probe bound to said nucleic acid molecule, thereby determining the presence or amount of the nucleic acid molecule in said sample.

Using the nucleotide sequences provided herein, probes can be constructed which are useful as reagents for detecting particular DNA sequences in a test sample. According to the present invention, a nucleic acid of the present invention can be labeled by any procedure known in the art to create a probe, e.g., by incorporation of radioactively labeled nucleotides.

The nucleic acids, derivatives and fragments thereof, that are included in the present invention can also be used for detecting or measuring expression products, for example, mRNAs complementary DNAs (cDNAs) and proteins derived from the present nucleic acids. Expression of mRNAs homologous to the nucleic acids of the present invention may be detected, for example, by Northern analysis, or by reverse transcription and amplification by PCR. Also contemplated are nucleic acid detection and quantification methods which employ signal moieties that are conjugated to nucleic acid probes. Nucleic acids in a sample can be immobilized on a solid support and hybridized to such probes. The signal moiety can be detected directly, for example by fluorescence. Alternatively, the signal moiety may be detected indirectly by its enzymatic activity, for example in an ELISA or other colorimetric assay.

In a preferred embodiment, expression of the nucleic acids disclosed in the present invention, in cancer cells is tested. Examples of cancer cells are melanoma cells.

Expression of any one of these genes is useful as an indication of whether the cancer will be susceptible to treatment using cytostatic drugs as specified in the context of the present invention. For example, where expression of any one of these genes, and/or different combinations thereof, is deregulated with respect to expression in drug-sensitive controls, resistance to the respective therapeutics will be expected and other therapeutics will be used instead.

In a further embodiment of the present invention, drug-resistant tumor cells are treated by administering an active compound / cytostatic drug as specified above in combination with the overexpression of either the nucleic acids or the antisense oligonucleotides. Overexpression is achieved by methods known to persons skilled in the art, preferably by transfecting cells with an expression vector carrying the nucleic acids as disclosed by GenBank accession numbers U63131, L11667, L48513, Z30093, X13293, L26081, M14200, X91171, D23662, L28824, L12401, D10522, M94345, AF063228, M19267, AF009615, U92436, U59831, U19251, Z50781, X79550, AF035812, X63468, M22919, L19605, X06820, M33336, S74017, L11066, U59877, M19645, X92669, D16581, M81601, V00530, D29013, M29874, L13740, M60974, X79683, U84388, M95809, D49547, L06623, D90209, J05428, K02770, X15880, X76771, X53280, M22760, U04520, L20298, Y14690, U13991, X06994, M22299, U44754, X58531, U84573, U11690, H45315, M11717, X85106, D50310, W68277, R09223, U36798, J04164, R76702, AB000584, R97220, M22976, AB023155, U79303, X67325, AB005047, AA410441, U46499, AB021288, U31345, M54968, M65212, J02611, X90858, U18728, M13755, H00952, H11608, X15729, H57310, M64925, L18967, AK000917, NM_018330, U90878, X02492, U50648, U09510, X01060, X57352, S45630, J04129, S40706, AF126743, NM_015894, NM_013451, AI624266, AL117608, NM_020182, NM_021216, AC007228, pos.66685-66811.

Preferably, the active compound used in combination with the overexpression of any of said nucleic acids is selected from the group consisting of cisplatin, etoposide and fotemustine.

Therefore, the present invention is also directed to a recombinant DNA and an expression vector which includes any one of the present nucleic acids operably linked to regulatory control nucleic acid which effects expression of the nucleic acid in a host cell. The present invention is further directed to a host cell which contains such a recombinant DNA or such an expression vector.

For the purpose of overexpression in drug-resistant tumor cells, the present nucleic acids can be placed in expression vectors capable of expressing an encoded protein, polypeptide or peptide. Nucleic acids encoding a desired protein or polypeptide are derived from cDNA or genomic clones using conventional restriction digestion or by synthetic methods, and are ligated into vectors from which they may be expressed. Vectors may, if desired, contain nucleic acids encoding portions of other proteins, thereby providing a fusion protein.

Expression vectors also include plasmids designed for the expression of proteins or polypeptides fused to or within bacterial phage coat proteins. The DNA encoding the desired protein or polypeptide, whether in a fusion, premature or mature form, may be ligated into expression vectors suitable for any host. The DNA encoding the desired polypeptide may also contain a signal sequence to permit secretion from the intended host. Both prokaryotic and eukaryotic host systems are contemplated.

The present invention is further directed to the use of these host cells to screen for compounds that may modulate the function of the said nucleic acids, fragments or derivatives thereof and/or said polypeptides, fragments or derivatives thereof, such as antisense RNAs, RNAi, morpholinos, peptide haptamers, antibodies or small inorganic or organic molecules.

The present invention is also directed to the use of these host cells to identify genes deregulated in repsonse to recombinant expression of the said nucleic acids, fragments or derivatives thereof and/or said polypeptides, fragments or derivatives thereof.

The present invention also contemplates a process for producing a recombinant proteins or immunogenic fragments thereof, encoded by the nucleic acids of the present invention. The process involves: a) culturing a host cell which contains an expression vector having one of the present nucleic acids in a culture medium under conditions suitable for expression of one of said recombinant proteins in the host cell, and b) isolating the recombinant protein from the host cell or the culture medium.

The present proteins, polypeptides and peptides, whether in a premature, mature or fused form, are isolated from lysed cells, or from the culture medium, and are purified to the extent needed for the intended use. One of skill in the art can readily purify these proteins, polypeptides and peptides by any available procedure. For example, purification may be accomplished by salt fractionation, size exclusion chromatography, ion exchange chromatography, reverse phase chromatography, affinity chromatography and the like.

Thus, a further object of the present invention are kits comprising at least one active compound, as described above, and at least one expression plasmid carrying one of the nucleic acids of the present invention, a fragment or derivative thereof. The said kits can be used for diagnostics or as a medicament for the treatment of cancer cells that are deregulated, as defined in the context of the present invention, in at least one of the nucleic acids of the present invention. Examples of such cancer types include neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, prostate carcinoma, and plasmocytoma.

The overexpression of the nucleic acids of the present invention in combination with at least one active compound is particularly suitable for the treatment of melanoma.

The present invention further contemplates antibodies which can bind to the present proteins, polypeptides and peptides. Such antibodies preferably bind to unique antigenic regions or epitopes in the present proteins, polypeptides and peptides.

Epitopes and antigenic regions useful for generating antibodies can be found within the present proteins, polypeptides or peptides by procedures available to one of skill in the art. For example, short, unique peptide sequences can be identified in the present proteins and polypeptides that have little or no homology to known amino acid sequences. Preferably the region of a protein selected to act as a peptide epitope or antigen is not entirely hydrophobic; hydrophilic regions are preferred because those regions likely constitute surface epitopes rather than internal regions of the present proteins and polypeptides. These surface epitopes are more readily detected in samples tested for the presence of the present proteins and polypeptides.

Peptides can be made by any procedure known to one of skill in the art, for example, by using in vitro translation or chemical synthesis procedures. Short peptides which provide an antigenic epitope but which by themselves are too small to induce an immune response may be conjugated to a suitable carrier. Suitable carriers and methods of linkage are well known in the art. Suitable carriers are typically large macromolecules such as proteins, polysaccharides and polymeric amino acids. Examples include serum albumins, keyhole limpet hemocyanin, ovalbumin, polylysine and the like. One of skill in the art can use available procedures and coupling reagents to link the desired peptide epitope to such a carrier. For example, coupling reagents can be used to form disulfide linkages or thioether linkages from the carrier to the peptide of interest. If the peptide lacks a disulfide group, one may be provided by the addition of a cysteine residue. Alternatively, coupling may be accomplished by activation of carboxyl groups.

The minimum size of peptides useful for obtaining antigen specific antibodies can vary widely. The minimum size must be sufficient to provide an antigenic epitope which is specific to the protein or polypeptide. The maximum size is not critical unless it is desired to obtain antibodies to one particular epitope. For example, a large polypeptide may comprise multiple epitopes, one epitope being particularly useful and a second epitope being immunodominant.

The antibody can be used as a diagnostic tool to determine the presence and amount of the polypeptides encoded by the nucleic acids of the present invention in cancer cells and comprising the aminoacid sequences AAB04798, AAA35731, AAC41995, CAA82909, CAA31655, AAA65938, AAA52171, CAA62596, BAA04889, AAA36526, NP_005563, BAA01392, AAA59570, AAC33443, AAA36771, AAC51766, AAC51182, AAC50660, AAC52045, CAA90644, CAA56093, AAB88513, CAA45068, AAA59893, AAA19734, CAA29968, AAB50921, AAB32188, NP_004125, AAB02832, AAA52614, CAA63356, BAA04013, AAA61138, CAA23789, BAA06099, AAA52144, AAA36763, AAA35863, CAA56130, AAC50952, AAA58399, BAA08495, AAA52342, BAA14234, AAA36793, AAA36106, CAA33889, CAA54166, CAA37375, AAA99220, AAF66217, AAA02868, CAA75002, AAA62230, CAA30052, AAB02844, AAC50358, CAA41418, AAB58363, AAA57004, NP_003061, AAA52697, CAA59427, BAA08849, NP_002520, NP_000921, AAB18673, AAA35494, NP061885, BAA19151, AAA52133, BAA76782, AAB50227, CAA47739, BAA25922, NP_064696, BAA35182, AAB60371, AAB41942, AAA68927, AAB59517, CAA62369, AAA85268, AAA36038, AAF19251, NP_005505, CAA33751, NP_003059, AAA60059, AAA20870, BAA91423, NP_060800, AAC05580, CAA26322, AAC50768, AAA86443, CAA25527, CAA40626, AAB23453, AAA60147, AAB22646, AAD38506, NP_056978, NP_038479, NP_000912, CAB56012, NP_064567, NP_067039, AAD23608.

The determination of the presence and amount of any one of these polypeptides in cancer cells is useful as an indication of whether the cancer will be susceptible to treatment with certain cytostatic drugs, in particular cisplatin, etoposide and fotemustine.

For example, where the amount of any one of these polypeptides, or combinations thereof, is deregulated with respect to the amount in drug-sensitive controls, resistance to e.g. cisplatin, etoposide, fotemustine and various other therapeutics which are substantially ineffective for treatment of DR cancers will be expected and other therapeutics will be used instead.

Methods to determine the presence and amount of a polypeptide in a given sample, using antibodies of the present invention are well known to the person skilled in the art. Briefly, a sample is provided, said sample is contacted with an antibody that immunospecifically binds to a given polypeptide and the presence or amount of antibody bound to said polypeptide is determined, whereby the presence or amount of polypeptides in said sample is determined. Methods to determine the amount and presence of polypeptides comprise, among others, Western blotting, immunoprecipitation, ELISA, and RIA.

### DESCRIPTION OF FIGURE AND TABLE

### Fig. 1.

Verification of differential expression of selected drug resistance candidate genes by Northern blot analysis. Radiolabeled cDNA fragments specific for the candidate genes depicted in rows A-O (nomenclature corresponds to official HGNC gene symbols) were hybridized to Northern blots containing total RNA. The RNAs were taken from different time points (t1 to t3) of cultivation of the resistant sublines and untreated MeWo. MeWo_{Eto1} time point t4 was either cultivated in presence (+) or absence (-) of etoposide. In addition, RNA from 24 h drug-treated MeWo cells (lane 8: C+, cisplatin; lane 12: E+, etoposide; lane 18: F+, fotemustine) and the respective untreated control (lane 4) was included in the analyses. The panels denoted as 28S show 28S rRNA bands of the RNA gels prior to blotting to demonstrate the RNA integrity and equal loading. Arrows at the right mark differently sized bands obtained for five of the genes. The respective sizes in kb are specified at the right of the panels.

### Table 1

Genes displaying differential expression in at least 2 drug resistant MeWo sublines. Column 1 lists official HGNC gene symbols, UniGene cluster numbers, IMAGE clone ID's or other identifiers of deregulated genes, together with NCBI LocusLink ID's (LL) and, if available, respective chromosomal localizations. Column 2 lists GenBank Accession Numbers representing mRNA sequences of the deregulated genes or, in the case of unknown genes, fragments thereof. Columns 3-5 exhibit the observed patterns of deregulation: Column 3, deregulation in MeWo_{Cis1} (c); Column 4, deregulation in MeWo_{Fote40} (f); Column 5, deregulation in MeWo_{Eto1} (e); +, upregulated; -, downregulated compared to the reference. If not consistently deregulated, numbers in columns 3 and 4 denote the single time points at which deregulation was observed. The definition of time points and criteria for the definition of differential expression in the resistant MeWo sublines are specified in the EXAMPLES section.

### EXAMPLES

### Genome-wide screen for differentially expressed genes in drug resistant melanoma cell lines

We used UniGene filter arrays with about 31.500 elements (RZPD p950 UniGene 1) for a genome-wide analysis of differentially expressed genes in drug resistant MeWo sublines. For the initial selection of drug resistance candidate genes, mRNA obtained from three different time points of cultivation under permanent selective pressure (t1 to t3, see materials and methods) was separately compared to an mRNA pool of three different time points (t1 to t3, see material and methods) of the long-term cultivated drug sensitive parental MeWo cell line. This was intended to select for stable alterations in the expression pattern of the drug resistant sublines and against genes that may be deregulated because of differences in the cell culture conditions. Genes showing distorted ratios of least 2.5 or 1/2.5, respectively, in at least two out of three comparisons were selected for further evaluation. This resulted in an initial number of 139 candidate genes, of which 87 were retrieved from MeWo_{Eto1}. From MeWo_{Cis1} and MeWo_{Fote40}, respectively, only 23 and 29 candidate genes were recovered. Seven genes were deregulated in two resistances whereas three genes displayed an overlap in all three sublines, so that, after elimination of these redundancies, a final set of 126 genes was obtained.

### Candidate gene evaluation

To further confirm and evaluate candidate genes, a drug resistance subarray with a final number of 1,321 elements was composed. These elements included the 126 candidate genes retrieved from the initial screen, 52 control elements for monitoring the hybridization quality, and 1,143 further elements from the RZPD Onco-library. The latter ones were included to specifically and more sensitively address known and putative cancer candidate genes in regard to their differential expression. For the complex hybridizations, we used the same mRNA populations as in the initial analyses, i. e., t1 to t3 for each resistant subline.

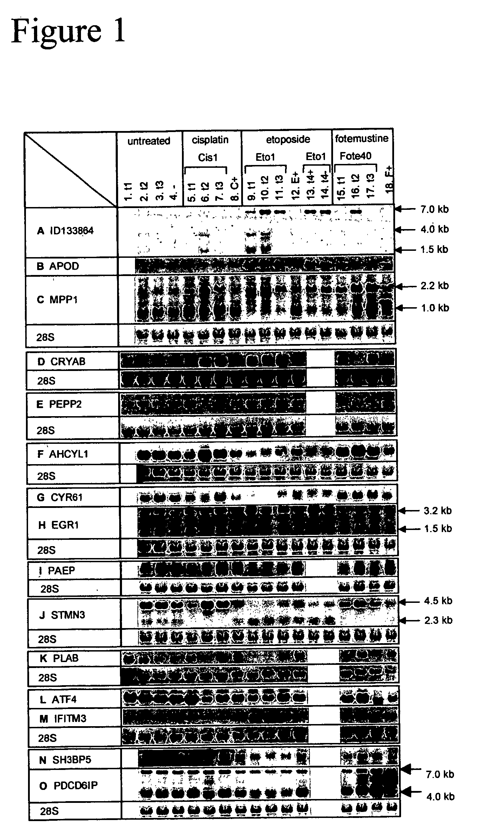

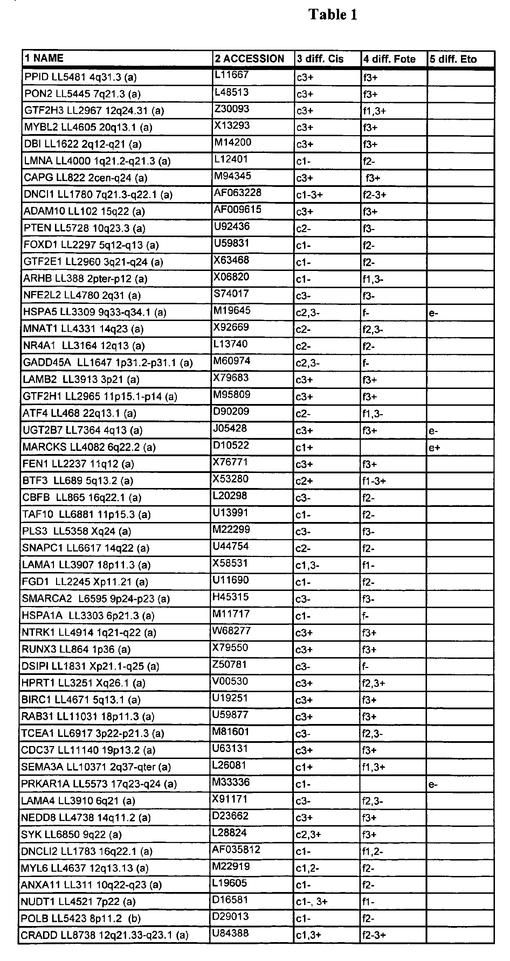

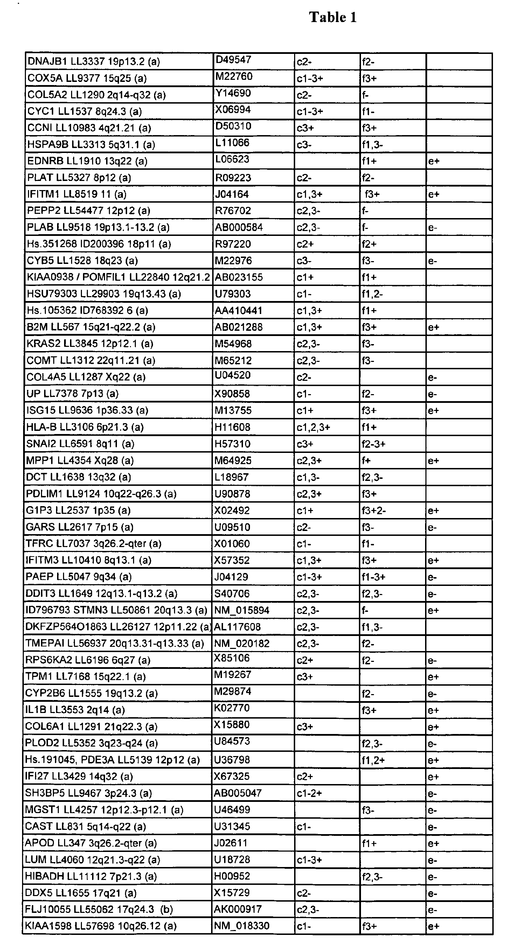

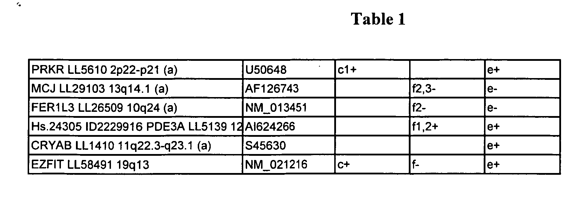

The subarray analyses indicated consistent changes in the expression patterns of all three time points investigated for MeWo_{Eto1}. Stable gene deregulation in MeWo_{Eto1} was further confirmed by analysis of mRNA of a fourth time point (t4). In contrast, the different time points of sublines MeWo_{Cis1} and MeWo_{Fote40} showed substantial variations. This was confirmed by two to three independent hybridization experiments as well as by Northern blot analyses of selected candidates (Fig. 1). We thus concluded that the differences in the stability of the transcriptomes had led to a differential efficacy in recovering candidates from the three sublines. In order to adjust to this phenomenon, we applied subline-specific criteria to identify primary candidates. For MeWo_{Eto1}, only genes showing consistent quantitative changes in all data points for three out of four analyzed time points were scored as primarily confirmed candidates. For MeWo_{Cis1} and MeWo_{Fote40}, genes displaying consistent changes in at least 5 out of 6 data points for t1 and t2 and in at least 3 out of 4 data points for t3, respectively, were scored as primarily confirmed candidates. Using these subline-specific criteria, 57 of the initial 126 genes (45%) were confirmed as being deregulated in at least one of the drug resistant sublines. Remarkably, 209 of the 1,143 (18%) included Onco library elements were suggested to be differentially expressed compared to the parental sensitive MeWo cell line. Thus, a total of 266 candidate genes resulted from these efforts. We selected a subset of 15 genes for further verification by Northern blot analyses. The candidates were analyzed in each of the 9 time points of the three drug resistant sublines, so that a total of 135 data points was investigated. For 110 of the 135 data points (81.4%) we obtained qualitatively concordant results (Fig. 1), confirming the applicability of the subline-specific criteria that were previously set.

For five of the 15 genes analyzed, we obtained more than one hybridizing band (Figure *1A, C, H, J, O).* The clone ID133864 mapped to two UniGene clusters, Hs.24305 and Hs.191045, and therefore was represented by two elements on the drug resistance subarray (Fig. 1*A).* By RT-PCR analyses, we identified the sequence of ID133864 as part of the 3'-UTR of the *PDE3A* gene. In Northern blot analysis using a *PDE3A* ORF sequence as a probe, a 7 kb transcript was detected (not shown) pointing to the 7 kb PDE3A transcript as the gene identified as differentially regulated in the etoposide and fotemustine resistant cell lines. In the case of *MPP1* (Fig. 1*C*), array data matched to the expression of the 2.2 kb variant, which is likely to represent the annotated mRNA (NM_002436). Both recently reported variants of the *PDCD6IP* gene displayed a downregulation in MeWo_{Eto1}, which was congruent with the array results (Figs. 1*O*). For *EGR1* (Fig. 1*H*), the expression pattern of the 3.2 kb variant correlated with the array data and the size of this variant corresponded to the annotated mRNA sequence (X52541). The *STMN3* gene (Fig. 1*J*) had been reported to give rise to a 2.3 kb transcript (NM_015894), which could be detected in Northern blot analysis. The quantitative changes in the levels of this transcript corresponded to the array data. For these five genes, other hybridizing bands may either represent alternative splice forms, polyadenylation variants or closely related genes. In the remaining 10 cases, the detected transcript sizes corresponded to the annotated mRNA sequences in GenBank.

### Genotype-transcriptome correlations

A comparison between the original CGH data and CGH data obtained from the resistant sublines at t3 indicated consistent genomic alterations on eight chromosomes. The specific genomic alterations allowed verifying the integrity of the respective sublines. We determined the cytogenetic localizations of the candidate genes by screening of NCBI (LocusLink, UniGene), RZPD and GDB databases. The differential expression of 28 genes could be correlated with gains and losses of the respective chromosomal regions.

### Cluster and Northern blot analyses reveal convergence of expression patterns in cisplatin and fotemustine resistance

Based on the 266 differentially expressed genes, we performed cluster analysis using the freely available GeneCluster and TreeView software to reveal common patterns of alterations. In accordance with the previous results, t1 to t4 of MeWo_{Eto1} located within one cluster. MeWo_{Cis1}t1 was related to MeWo_{Eto1} according to the clustering whereas MeWo_{Fote40}t1 and t2 represented a distinct entity. However, MeWo_{Cis1}t2/t3 and MeWo_{Fote40}t3 deviated from their original patterns and appeared to finally converge in regard to their expression patterns. We verified these results by Northern blot analysis of selected candidates. Twelve of 15 genes showed consistent quantitative changes over all MeWo_{Eto1} time points analyzed (for example ID133864, *APOD, CRYAB,* and *AHCYL1* in Fig. 1) Six of the 15 genes displayed convergence in their expression levels at the later time points when comparing MeWo_{Cis1} and MeWo_{Fote40} (see ID133864, *APOD, CRYAB, PEPP2, PLAB, IFITM3* in Fig. 1). *CRYAB,* one of the genes represented and up regulated in both groups has been related to the repression of apoptosis.

### Evaluation of short-term response and cross-resistance candidate genes

In order to distinguish between stably deregulated genes and genes deregulated due to the presence of the drugs, we conducted a series of further experiments. At first, we included RNA samples of the parental sensitive MeWo cell line treated for 24 h with cisplatin and fotemustine in the Northern blot analyses. Secondly, we additionally carried out Northern blot and subarray analyses with 24 h etoposide induced MeWo cells as well as with MeWo_{Eto1} omitting the etoposide from the culture medium. The latter subarray analyses indicated no major influence of etoposide on the overall expression pattern in MeWo_{Eto1}, as suggested by clustering. In contrast, the patterns observed for short-term treated MeWo substantially differed from both untreated MeWo and drug resistant MeWo_{Eto1}. These data suggested, that genes deregulated due to the short-term response to the presence of the drug are represented only to a minor extent in the candidate panel. Basically, these data were confirmed by the Northern blot analyses. Twelve of the fifteen genes showing quantitative differences in the resistant sublines were not responsive to short-term drug treatment. Remarkably, however, three of the fifteen genes *(AHCYL1, CYR61* and the 4.5 kb variant of *STMN3)* displayed a down-regulation in the course of the short-term response to all three DNA-damaging drugs. The down-regulation of all three genes became manifested in the long-term resistant cell line MeWo_{Eto1,} and two of these *(CYR61,* and the 4.5 kb transcript of *STMN3)* were also transiently down regulated at one of the time points in MeWo_{Cis1}. The transcriptional repression of the three genes in MeWo_{Eto1} was maintained even in the absence of etoposide (Fig. 1). Moreover, two of these short-term response genes mapped close to a region at chromosome 1 identified as genomic loss in MeWo_{Eto1} by CGH. *CYR61* was located at 1p31-p22 (1p22.3 according to GeneCards), whereas *AHCYL1* had been mapped to 1p12.

In order to identify potential cross-resistance candidates, we compared the data obtained for the three different drug-resistant sublines from the subarray analyses. This revealed substantial overlaps in the differential expression of genes during acquired drug resistance. In total, 110 differentially expressed genes showed overlaps in their deregulation. Fourteen of these were found to be deregulated in all three drug-resistant cell lines.

### Arrangement according to common pathways points to frequent deregulation ofapoptosis-related genes

Subsets of the deregulated genes could be identified to belong to common pathways. For example, six genes, *G1P3, ISG15, IFI27, IFITM1, IFITM3,* and *PRKR,* represent a group of interferon (IFN) inducible genes. These genes were highly over expressed in MeWo_{Eto1} and in MeWo_{Cis1}t1, which is in agreement with the data obtained for a paclitaxel resistant subline of the breast cancer cell line MCF-7. However, whereas these changes were persistent in MeWo_{Eto1}, these genes again displayed the lower levels of the parental MeWo cells in MeWo_{Cis1}t2/t3. Secondly, several stress inducible genes, e. g., *PLAB, HSPA5, HSPA9B, DDIT3,* and *GADD45A* were often down regulated in the drug resistant sublines, to the major part in MeWo_{Eto1} (Fig. 1). Remarkably, 12 genes belonging to apoptosis-related pathways were commonly found to be deregulated, as for example *CRYAB, DFFA, PDCD6IP* or *SH3BP5.*

### MATERIALS AND METHODS

### Cell culture and cell lines

Drug resistant derivatives of MeWo had been selected as described in the prior art. MeWo cells were grown in RPMI medium supplemented with 10% fetal calf serum, the antibiotics penicillin and streptomycin (100 U/ml each), and L-glutamine (2 mM). Stable chemoresistant MeWo sublines were grown in supplemented RPMI with the respective drugs: MeWo_{Cis1} with additional cisplatin (1 µg/ml), MeWo_{Eto1} with additional etoposide (1 µg/ml) and MeWo_{Fote40} with additional fotemustine (40 µg/ml). In general, cells were grown to 80-90% confluence and then harvested for RNA and DNA isolation. Drug treatment of MeWo cells occurred at about 80% confluence for 24 hrs with the concentrations depicted above. In parallel, MeWo control cells were cultivated for the same time without drugs. For the determination of the effects of etoposide treatment on gene expression in MeWo_{Eto1}, we grew cells with and without etoposide supplementation. For the array hybridizations, RNA was prepared from at least three different time points (t1-t3) for each of the cell lines. The cell lines were reconstituted from frozen stocks collected during continuous growth over a period of about 2.5 years. For the sensitive MeWo reference, the time point t1 represents the parental cells from which the drug resistant sublines were established. The time point t2 and t3 represent cells, which were continuously cultured for additional 23 and 42 months, respectively. For all drug resistant sublines, t1 denotes the time point of the definition of drug resistance. The nomenclature for further time points of drug resistant sublines is as follows: MeWo_{Cis1}, t2: 12 months, and t3: 31 months; MeWo_{Fote40}, t2: 11 months, and t3: 30 months; MeWo_{Eto1}, t2: 3 months, t3: 22 months, and t4: 46 months after definition of drug resistance. In CGH analyses, t1+ of MeWo_{cis1} and MeWo_{Fote40} corresponds to t1 + 8-9 months.

### cDNA arrays

The genome-wide cDNA array (RZPD p950 UniGene1) was manufactured as described previously. The drug resistance subarray was composed of the 126 candidate genes retrieved from the initial screening of the UniGene array, 52 control genes, and 1,143 genes amplified from the RZPD Onco-library. The entire RZPD Onco-library comprises about 2,800 cancer candidate genes. For the subarray, cluster-specific oligonucleotides (based on the NCBI UniGene built 90, September 1999) were selected for the amplification of 300 bp cluster segments that are free of low complexity regions and corresponding to 3' termini of mRNA molecules. For the PCR amplification we used pools of plasmid DNA (1 ng per reaction, 96-384 different template species per pool) as templates. PCR was performed in 96-well plates for 30 cycles (20 s 94 °C, 15 s 46°C, 30 s 72°C). These elements were spotted in a 3 x 3 pattern on a 7 x 10 cm nylon membrane. All PCR products were spotted in duplicate as described before to provide internal controls. For image processing, each block was provided with a guide spot that contains heterologous DNA from the bacterial kanamycin resistance gene.

### Complex cDNA hybridization

mRNA was isolated using the RNEasy kit and the Oligotex mRNA kit (Qiagen) according to the manufacturer's recommendations. The mRNA integrity was confirmed by electrophoresis in denaturing 1% agarose gels with formaldehyde, blotting to Hybond N+ Nylon membranes (Amersham-Pharmacia Biotech) and hybridization with radiolabeled dT₁₈V oligonucleotide. The generation of complex cDNA probes was performed as described previously. After purification, the specific activity of the probes was determined and volumes of the probes were adjusted to ascertain equivalent conditions for the array hybridizations. The arrays were hybridized under the conditions described before. UniGene arrays were hybridized at 65 °C whereas the drug resistance subarray was hybridized at 63 °C to adjust for the smaller average fragment length.

### Image acquisition and analysis of array hybridizations

For RZPD UniGene 1 arrays, image acquisition, grid alignment and spot quantification was performed as described previously. Spot by spot comparison, normalization, and determination of differential expression was carried out using fdiffs, an algorithm developed by T. Beissbarth (Theoretical Bioinformatics, DKFZ) based on the matlab package (MathWorks, Natick, MA, USA). For the analysis of the drug resistance subarrays we used ArrayVision Software (Imaging Research). Here, after spot finding using an automated algorithm, signal intensities were calculated as mean pixel values minus a regional background calculated for each 3 x 3 spot group, respectively. For array comparisons, signal intensities were then normalized to the mean of all human cDNA containing spots on one filter, and expression ratios for single spots were calculated. Ratios of 2.5 and 1/2.5 were set as thresholds to identify differentially expressed genes from the UniGene arrays. For the drug resistance subarray, we used ratios of 1.5 and 1/1.5 as the threshold values to adapt to the decrease in the average size of the array elements.

### Northern blot analyses

Fifteen µg total RNA was separated by electrophoresis in 1% agarose gels under denaturing conditions in the presence of 2.2 M Formaldehyde, and stained with SYBR Green II (FMC) for visualization under UV-light. RNA was transferred to Hybond N+ Nylon membranes (Amersham-Pharmacia Biotech) by capillary transfer overnight and subsequently immobilized by UV cross-linking. Hybridizations were performed with ³²P labeled cDNA probes in 500 mM Na₂HPO₄, pH 7.2, 7% SDS and 10 mM EDTA at 65 °C overnight. After washing (2 x 10 minutes in 0.5 x SSC, 0.1% SDS at 65 °C) membranes were exposed to X-ray films and imaging plates. In the latter case image acquisition was done using a Fuji FLA3000 phosphorimager and AIDA software.

### Comparative genomic hybridization

Cells were lysed by incubation in 50mM Tris, 100mM EDTA and 200mM NaCl (pH 9) with 1% SDS and 0.5 mg/ml Proteinase K at 37 °C overnight. Genomic DNA was purified by a standard phenol chloroform extraction procedure and subsequent ethanol precipitation. Genomic DNA was reconstituted in 10 mM Tris, 1 mM EDTA, pH 7.5. Probe preparation, hybridization, image acquisition, and analysis of CGH were performed as described previously.

## Claims

1. A nucleic acid molecule encoding a human protein, which is deregulated in drug-resistant tumor cells in response to drug exposure, wherein said nucleic acid comprises a coding sequence from the group of sequence identification numbers: U63131, L11667, L48513, Z30093, X13293, L26081, M14200, X91171, D23662, L28824, L12401, D10522, M94345, AF063228, M19267, AF009615, U92436, U59831, U19251, Z50781, X79550, AF035812, X63468, M22919, L19605, X06820, M33336, S74017, L11066, U59877, M19645, X92669, D16581, M81601, V00530, D29013, M29874, L13740, M60974, X79683, U84388, M95809, D49547, L06623, D90209, J05428, K02770, X15880, X76771, X53280, M22760, U04520, L20298, Y14690, U13991, X06994, M22299, U44754, X58531, U84573, U11690, H45315, M11717, X85106, D50310, W68277, R09223, U36798, J04164, R76702, AB000584, R97220, M22976, AB023155, U79303, X67325, AB005047, AA410441, U46499, AB021288, U31345, M54968, M65212, J02611, X90858, U18728, M13755, H00952, H11608, X15729, H57310, M64925, L18967, AK000917, NM_018330, U90878, X02492, U50648, U09510, X01060, X57352, S45630, J04129, S40706, AF126743, NM_015894, NM_013451, AI624266, AL117608, NM_020182, NM_021216, AC007228, pos.66685-66811, or a derivative or fragment thereof.

2. A nucleic acid molecule according to claim 1, the deregulated expression of which is due to either a loss or a gain of a certain chromosomal region or by other causes leaving the respective chromosomal region unaffected.

3. A nucleic acid molecule according to claims 1 and 2, wherein the drug causing deregulation of said nucleic acid molecules is selected from the group consisting of (i) antimetabolites, preferably cytarabine, fludarabine, 5-fluoro-2'-deoxyuiridine, gemcitabine, hydroxyurea or methotrexate; (ii) DNA-fragmenting agents, preferably bleomycin, (iii) DNA-crosslinking agents, preferably chlorambucil, cisplatin, fotemustine, cyclophosphamide or nitrogen mustard; (iv) intercalating agents preferably adriamycin (doxorubicin) or mitoxantrone; (v) protein synthesis inhibitors, preferably L-asparaginase, cycloheximide, puromycin or diphteria toxin; (vi) topoisomerase I poisons, preferably camptothecin or topotecan; (vii) topoisomerase II poisons, preferably etoposide (VP-16) or teniposide; (viii) microtubule-directed agents, preferably colcemid, colchicine, paclitaxel, vinblastine or vincristine; (ix) kinase inhibitors preferably flavopiridol, staurosporin, STI571 (CPG 57148B) or UCN-01 (7-hydroxystaurosporine); (x) miscellaneous investigational agents preferably thioplatin, PS-341, phenylbutyrate, ET-18-OCH₃, or farnesyl transferase inhibitors (L-739749, L-744832); polyphenols preferably quercetin, resveratrol, piceatannol, epigallocatechine gallate, theaflavins, flavanols, procyanidins, betulinic acid and derivatives thereof; (xi) hormones preferably glucocorticoids or fenretinide; (xii) hormone antagonists, preferably tamoxifen, finasteride or LHRH antagonists, in particular from the group consisting of cisplatin, etoposide and fotemustine.

4. A polypeptide encoded by at least of the nucleic acids according to claim 1 and comprising an aminoacid sequence from the group of sequence identification numbers AAB04798, AAA35731, AAC41995, CAA82909, CAA31655, AAA65938, AAA52171, CAA62596, BAA04889, AAA36526, NP_005563, BAA01392, AAA59570, AAC33443, AAA36771, AAC51766, AAC51182, AAC50660, AAC52045, CAA90644, CAA56093, AAB88513, CAA45068, AAA59893, AAA19734, CAA29968, AAB50921, AAB32188, NP_004125, AAB02832, AAA52614, CAA63356, BAA04013, AAA61138, CAA23789, BAA06099, AAA52144, AAA36763, AAA35863, CAA56130, AAC50952, AAA58399, BAA08495, AAA52342, BAA14234, AAA36793, AAA36106, CAA33889, CAA54166, CAA37375, AAA99220, AAF66217, AAA02868, CAA75002, AAA62230, CAA30052, AAB02844, AAC50358, CAA41418, AAB58363, AAA57004, NP_003061, AAA52697, CAA59427, BAA08849, NP_002520, NP_000921, AAB18673, AAA35494, NP061885, BAA19151, AAA52133, BAA76782, AAB50227, CAA47739, BAA25922, NP_064696, BAA35182, AAB60371, AAB41942, AAA68927, AAB59517, CAA62369, AAA85268, AAA36038, AAF19251, NP_005505, CAA33751, NP_003059, AAA60059, AAA20870, BAA91423, NP_060800, AAC05580, CAA26322, AAC50768, AAA86443, CAA25527, CAA40626, AAB23453, AAA60147, AAB22646, AAD38506, NP_056978, NP_038479, NP_000912, CAB56012, NP_064567, NP_067039, AAD23608, or a fragment or derivative thereof.

5. An activator which is capable of increasing the expression of at least one nucleic acid, a fragment or derivative thereof.

6. An inhibitor which is capable of decreasing the expression of at least one nucleic acid, a fragment or derivative thereof.

7. A pharmaceutical composition comprising at least one nucleic acid according to claim 1, a fragment or derivative thereof, optionally in combination with at least one active compound.

8. A pharmaceutical composition comprising at least one polypeptide according to claim 4, a fragment or derivative thereof, optionally in combination with at least one active compound.

9. A pharmaceutical composition according to claims 7 and 8, wherein the active compound is a cytostatic drug.

10. A pharmaceutical composition according to any of claims 7 to 9, wherein the cytostatic drug is selected from the group consisting of (i) antimetabolites, preferably cytarabine, fludarabine, 5-fluoro-2'-deoxyuiridine, gemcitabine, hydroxyurea or methotrexate; (ii) DNA-fragmenting agents, preferably bleomycin, (iii) DNA-crosslinking agents, preferably chlorambucil, cisplatin, fotemustine, cyclophosphamide or nitrogen mustard; (iv) intercalating agents preferably adriamycin (doxorubicin) or mitoxantrone; (v) protein synthesis inhibitors, preferably L-asparaginase, cycloheximide, puromycin or diphteria toxin; (vi) topoisomerase I poisons, preferably camptothecin or topotecan; (vii) topoisomerase II poisons, preferably etoposide (VP-16) or teniposide; (viii) microtubule-directed agents, preferably colcemid, colchicine, paclitaxel, vinblastine or vincristine; (ix) kinase inhibitors preferably flavopiridol, staurosporin, STI571 (CPG 57148B) or UCN-01 (7-hydroxystaurosporine); (x) miscellaneous investigational agents preferably thioplatin, PS-341, phenylbutyrate, ET-18-OCH₃, or farnesyl transferase inhibitors (L-739749, L-744832); polyphenols preferably quercetin, resveratrol, piceatannol, epigallocatechine gallate, theaflavins, flavanols, procyanidins, betulinic acid and derivatives thereof; (xi) hormones preferably glucocorticoids or fenretinide; (xii) hormone antagonists, preferably tamoxifen, finasteride or LHRH antagonists, in particular from the group consisting of cisplatin, etoposide and fotemustine.

11. The use of at least one nucleic acid according to claim 1 and / or at least one polypeptide according to claim 4, optionally in combination with at least one active compound, for the manufacture of a medicament for the treatment of cancer.

12. The use according to claim 11, wherein the cancer to be treated is selected from the group consisting of neuroblastoma, intestine carcinoma preferably rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors preferably glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, prostate carcinoma and plasmocytoma, in particular melanoma.

13. A medicament for the treatment of cancer, comprising a nucleic acid and / or a polypeptide and / or an activator and / or an inhibitor as claimed in claims 1, 4, 5 and 6, respectively, and a pharmaceutically acceptable carrier.

14. A diagnostic kit for the detection of deregulated expression in said cancer cells, comprising at least one of the nucleic acids of claim 1.

15. A method for the diagnosis or monitoring of drug-resistance in a human using the kit according to claim 14, the method comprising: (i) contacting an appropriate sample of the respective human with a probe that binds to said nucleic acid molecules, and (ii) determining the presence or amount of the probe bound to said nucleic acid molecule, thereby determining the presence or amount of the nucleic acid molecule in said sample.

16. A recombinant DNA comprising at least one of the nucleic acids according to claim 1, operable linked to regulatory control nucleic acid sequences which can affect expression of said nucleic acid sequences in a host cell.

17. An expression vector or plasmid comprising the recombinant DNA according to claim 16.

18. A host cell comprising the expression vector of claim 17, wherein the host cell is a eukaryotic or prokaryotic cell.

19. The use of a host cell according to claim 18, to screen for modulators of the recombinant DNA according to claim 16 and to identify deregulated genes.

20. A method of producing recombinant proteins or immunogenic fragments thereof, encoded by the recombinant DNA according to claim 16, which process comprises (i) culturing a host cell in a culture medium suitable for the expression of said proteins or immunogenic fragments thereof, and (ii) recovering said recombinant proteins or immunogenic fragments thereof from said host cell or said culture medium.

21. The use of an expression vector according to claim 17, optionally in combination with an active compound for the manufacture of a medicament for the treatment of melanoma and related cancers.

22. The use according to claim 21, wherein the active compound is selected from the group consisting of (i) antimetabolites, preferably cytarabine, fludarabine, 5-fluoro-2'-deoxyuiridine, gemcitabine, hydroxyurea or methotrexate; (ii) DNA-fragmenting agents, preferably bleomycin, (iii) DNA-crosslinking agents, preferably chlorambucil, cisplatin, fotemustine, cyclophosphamide or nitrogen mustard; (iv) intercalating agents preferably adriamycin (doxorubicin) or mitoxantrone; (v) protein synthesis inhibitors, preferably L-asparaginase, cycloheximide, puromycin or diphteria toxin; (vi) topoisomerase I poisons, preferably camptothecin or topotecan; (vii) topoisomerase II poisons, preferably etoposide (VP-16) or teniposide; (viii) microtubule-directed agents, preferably colcemid, colchicine, paclitaxel, vinblastine or vincristine; (ix) kinase inhibitors preferably flavopiridol, staurosporin, STI571 (CPG 57148B) or UCN-01 (7-hydroxystaurosporine); (x) miscellaneous investigational agents preferably thioplatin, PS-341, phenylbutyrate, ET-18-OCH₃, or farnesyl transferase inhibitors (L-739749, L-744832); polyphenols preferably quercetin, resveratrol, piceatannol, epigallocatechine gallate, theaflavins, flavanols, procyanidins, betulinic acid and derivatives thereof; (xi) hormones preferably glucocorticoids or fenretinide; (xii) hormone antagonists, preferably tamoxifen, finasteride or LHRH antagonists, in particular from the group consisting of cisplatin, etoposide and fotemustine.

23. A kit, comprising at least one active compound, as described above, and an expression vector according to claim 17 for the treatment of cancer cells which are deregulated in at least one of the nucleic acids according to claim 1.

24. Antibodies that immunospecifically bind to the polypeptides according to claim 4.

25. A method for determining the presence and amount a polypeptide in a human according to claim 4, the method comprising (i) contacting an appropriate sample with an antibody that binds immunospecifically to one of said polypeptides, and (ii) determining the presence or amount of antibody bound to said polypeptide, thereby determining the presence or amount of polypeptide in said sample.

26. A diagnostic kit comprising at least one antibody according to claim 24 to determine the presence and amount of the polypeptides according to claim 4 in drug-sensitive cancer cells.
